# EUROPEAN PATENT APPLICATION

(11) **EP 1 743 522 A1**
(43) Date of publication of application: **17.01.2007**
(21) Application number: 05076610.4
(22) Date of filing: 13.07.2005
(51) Int. Cl.: A01K 67/027, A61P 3/10

(54) **An animal model for type II diabetes mellitus and syndrome x**

(71) Applicant: ID-Lelystad, Instituut voor Dierhouderij en Diergezondheid B.V., 8219 PH Lelystad (NL)
(72) Inventor: Koopmans, Sietse Jan, 2376 AK Nieuwe Wetering (NL); Mroz, Zdzislaw, 8223 GS Lelystad (NL)
(74) Representative: Winckels, Johannes Hubertus F.

(57) **Abstract**

The invention provides a method for generating a type II diabetes mellitus and/or Syndrome X model in pigs. A method of the invention comprises partially destructing pancreatic beta-cells in pigs. From said pigs, a pig is preferably selected that comprises a fasting plasma glucose level higher than 6 mmol/L. The invention further provides a pig according to the invention and uses thereof.

## Description

The invention relates to the field of endocrinology and metabolism. Specifically the invention relates to an animal model for type II diabetes mellitus and/or Syndrome X, and uses thereof.

Diabetes is considered a growing epidemic by the World Health Organisation. Worldwide the prevalence of diabetes is between 2-7% of the population, increasing with age to between 10-14% of the population aged over 40 years of age. Diabetes mellitus can cause multiple complications, which makes it one of the leading causes of morbidity and mortality in the United States. In 1994 already 1 of every 7 healthcare dollars in the United States was spent on patients with diabetes mellitus. Estimates in many other countries amounted to 5-10% of the healthcare budget spent on patients with diabetes in the '90 ties. Since then the prevalence of diabetes has rapidly increased. Type II diabetes mellitus accounts for about 85-90% of all patients with diabetes. Type II diabetes mellitus is also the type of diabetes that is mainly responsible for the rapidly increasing prevalence of diabetes.

Diabetes is a metabolic disorder in which the glucose homeostasis is disturbed. Insulin is the hormone that keeps blood sugar levels within the normal range in healthy subjects. In patients with diabetes the glucose level in the blood is too high for most of the time. There are two main types of diabetes. Type I diabetes usually occurs in young people and is caused by failure of the pancreas to produce insulin. Type II diabetes used to be a disorder predominantly occurring amongst the middle-aged or elderly. Nowadays, however, the mean age at which this disorder occurs has lowered significantly. Even in teenagers diabetes type II is not a rare phenomenon anymore. Type II diabetes is mainly characterised by a resistance of the body to the action of insulin. Resistance to the actions of insulin in muscle, fat, and liver results in decreased glucose transport in muscle, elevated hepatic glucose production and increased breakdown of fat. Type II diabetes is caused by a combination of genetic and environmental factors. Included in these environmental factors is what is called a diabetogenic lifestyle. That is a lifestyle characterized by consumption of excessive calories, inadequate caloric expenditure and obesity. Although classic symptoms of diabetes are polyuria, polydipsia, polyphagia and weight loss, multiple other symptoms are also associated with diabetes. Diverse symptoms are due to complications of diabetes, those complications can be divided into three main categories. The first category consists of acute complications due to low or high blood sugar leading to ketoacidosis. Acute complications form a more important category in type I diabetes then in type II diabetes. The second category comprises microvascular complications such as retinopathy, nephropathy and neuropathy. The third category entails macrovascular complications comprising heart disease, stroke and peripheral vascular disease of which the latter can result in amputation. Type II diabetes is often diagnosed late, between onset and diagnosis lies on average a period of 7 years. The late diagnosis is often due to the fact that the symptoms of diabetes type II at the onset of the disease frequently manifest themselves mildly and there may not be many symptoms yet. But even when diabetes type II appears to be asymptomatic the present hyperglycemia and insulin resistance can already be affecting the individual.

As already mentioned, one of the environmental risk factors of diabetes mellitus is obesity. The prevalence of obesity is increasing rapidly in all age groups in most EU-countries. It currently affects at least 10-50% of the adult population in the EU, running up to percentages already established in the United States, about 60%. With the increased prevalence of obesity comes an increased risk of serious co-morbidities as said diabetes, cardiovascular disease, certain cancers and reduced life expectancy. Obesity is also a factor in a disorder called Syndrome X. Other names of the same condition are amongst others Metabolic Syndrome and Dysmetabolic Syndrome. Syndrome X is characterised by glucose intolerance in various degrees and two or more of the following symptoms: obesity, central obesity, dyslipidemia (elevated triglycerides, elevated low density lipoprotein (LDL) or reduced high density lipoprotein level, HDL), arterial hypertension or microalbuminuria. Just like in diabetes mellitus type II the cause of Syndrome X is a combination of genetic factors and environmental factors. Syndrome X has an increasing prevalence and while formerly being a disease of the middle-aged and elderly it is now also described as presenting itself in young adults, adolescents and children.

The invention provides a method for generating a type II diabetes mellitus and/or Syndrome X model in pigs, comprising partially destructing pancreatic beta-cells in pigs and selecting from said pigs a pig that comprises a fasting plasma glucose level higher than 6 mmol/L. Pigs having said plasma glucose level are a good model for at least syndrome X. Syndrome X is generally seen as a pre-stage of type II diabetes mellitus. An early sign of Syndrome X is a fasting plasma glucose level higher than 6 mmol/L. In the present invention the pigs are preferably selected on the basis of this criterion. However, pigs may be selected for other criteria of Syndrome X. Such selection criteria are also within the scope of the present invention when they inherently result in a selected pig having a fasting plasma glucose level higher than 6 mmol/L. These selection criteria preferably encompass selecting a pig exhibiting whole body insulin resistance, hepatic insulin resistance and/or plasma triglycerides levels in a fasting and/or postprandial phase elevated by at least a factor of 2 when compared to an untreated pig. In a preferred embodiment these other criteria are combined with selecting a pig that exhibits a non ketotic status or maintenance of growth in the absence of insulin therapy. Selecting for these other criteria result in a pig having a fasting plasma glucose level higher than 6 mmol/L. In a preferred embodiment the pigs are selected by measuring fasting plasma glucose levels or an equivalent thereof, and selecting a pig having a fasting plasma glucose level higher than 6 mmol/L. Pigs with a fasting plasma glucose level higher than 6 mmol/L are a good model for early Syndrome X, and/or suitable for developing a model for Syndrome X. When it is desired to start with pigs that are further on the way toward type II diabetes mellitus, it is preferred to select pigs comprising a fasting plasma glucose level of 10 mmol/L or higher. For Syndrome X the pigs preferably have a fasting plasma glucose level between 8 and 12 mmol/, more preferably between 9 and 11 mmol/L, and particularly preferred about 10 mmol/L.
Type II diabetes mellitus in humans can result (in untreated patients) in very high fasting plasma glucose levels. When selecting pigs for type II diabetes mellitus it is preferred to select pigs having a fasting plasma glucose level higher than 15 mmol/L. Preferably between 15 and 40 mmol/L, more preferably between 15 and 25 mmol/L. These plasma levels can also be selected on the basis of the other criteria mentioned above, however, preferably the pigs are selected on the basis of fasting plasma glucose levels. Alternatively a pig is selected that comprises an elevated fasting plasma level of a functional equivalent of glucose as compared to a fasting plasma level of said functional equivalent of a healthy pig. A functional equivalent of glucose is herein defined as a compound, the fasting plasma level of which is indicative for the absence or presence of diabetes mellitus type II or Syndrome X. Said functional equivalent for instance comprises a monosaccharide, glycosylated haemoglobin or fructosamine. For instance, according to the invention the fasting plasma level of fructosamine in healthy pigs is about 220 µmol/L, whereas the fasting plasma level of fructosamine in diabetic pigs is about 550 µmol/L. Hence said plasma level is indicative for diabetes.

The invention further provides a method for generating a type II diabetes mellitus and/or Syndrome X model in pigs, comprising partially destructing pancreatic beta-cells in pigs and selecting from said pigs a pig that shows at least one of the features selected from group 1 and at least one of the features selected from group 2 after said partial destruction of said pancreatic beta-cells, wherein group 1 comprises: whole body insulin resistance, hepatic insulin resistance, plasma triglyceride levels in a fasting and/or postprandial phase elevated by at least a factor 2 when compared to an untreated pig or a fasting plasma glucose level higher than 10 mmol/L and wherein group 2 comprises: a non-ketotic status or maintenance of growth without insulin therapy. Destructing is defined as rendering functionally inactive which can for example be achieved by removal, chemical destruction or blockage. Whole body insulin resistance is defined as sub-normal responsiveness of cells in various parts of the body upon exposure to insulin. The sub-normal responsiveness results in lower than normal whole body glucose uptake upon insulin stimulation. When stimulated by insulin, the whole body glucose uptake is at least 2-fold, preferably 3-fold, lower in diabetic than in normal, untreated, pigs. Hepatic insulin resistance is defined as sub-normal responsiveness of cells of the liver upon exposure to insulin. The sub-normal responsiveness manifests as a reduced inhibition of hepatic glucose production upon exposure to insulin compared to normal, untreated, pigs. The hepatic glucose production upon exposure to insulin is 1.5-fold, preferably 2-fold, greater in diabetic than in normal, untreated, pigs.

Surprisingly, we found that in the present invention insulin resistance remains stable. This is for example useful when investigating long-term effects of pharmaceutical compositions or nutrition compounds. In the present invention insulin resistance remains stable for at least one week, preferably during two or more weeks. In the present invention the severity of insulin resistance shows little fluctuation upon acute changes in plasma glucose concentrations, preferably not more than 25%, more preferably not more than 20%.

In a preferred embodiment the invention provides a method wherein said pig shows a fasting plasma glucose level higher than 10 mmol/L. Fasting is defined as not having eaten food for a period of at least 6 hours. Preferably, said pig shows a concomitant plasma insulin concentration which is higher, preferably 2-fold higher, than in normal, untreated, pigs. Establishment whether a pig is in a ketotic or a non-ketotic state can be achieved by detection of ketones in the urine of that pig. No or trace amounts of ketones are found in the urine of a non-ketotic pig, wherein trace amounts are in the range of 0-0.7 mmol/L. In the urine of a ketotic pig, ketones are found in amounts of at least 0.7 mmol/L. A patient with diabetes mellitus type I cannot maintain growth without insulin therapy. A patient with diabetes type II, however, is often able to maintain growth without insulin therapy. Thus in a preferred embodiment said insulin deficient pig is capable of maintaining growth. In another preferred embodiment a pig shows at least the following features: plasma triglyceride levels in a fasting and/or postprandial phase elevated by at least a factor 2 when compared to an untreated pig and a non-ketotic status.

In the present invention, at least some of the disadvantages of classical animal models for diabetes mellitus type II have been overcome. Thereto the invention provides an animal model in a pig of a breed that is capable of reaching human adolescent or human mature weight, preferably defined by a weight of 70-150kg at 5-9 months of age. Said pig is capable of representing a healthy weight as well as representing an overweight or even an obese individual. A pig of the invention has an anatomical, physiological and metabolic resemblance to human. Marshall (1979) has described chronic diabetes in a miniature pig. Miniature pigs are inbred. I.e. homozygous for at least 90% of all genetic markers and preferably at least 95% all genetic markers. Inbred lines are generally obtained and/or maintained by interbreeding between parents and/or siblings and/or equivalents thereof. An equivalent of a sibling is a pig having a similar genetic background. The present invention preferably uses an outbred population of pigs. An outbred population in the present invention is characterised in that the pigs are heterozygous for at least 50% and preferably at least 70% of all genetic markers. Outbred populations are typically obtained by breeding not related pigs. An outbred population that is within the scope of the present invention is progeny of a cross between two inbred lines that in the F1 result in a population of pigs that are heterozygous for at least 50% and preferably at least 70% of all genetic markers. However, in a preferred embodiment of the invention, the population of pigs for creating the model is outbred on the basis that none of its ancestors is an inbred line. Use of an outbred population brings diversity in the response with it. This is a preferred feature of the present invention. In a preferred embodiment said outbred population of pigs comprises crossbred pigs. Preferably a cross-breed of Yorkshire x Landrace.
An outbred population is a better model for representing a human being with a complex disease as Syndrome X or diabetes mellitus type II. A model of the present invention has a further advantage in that it reaches a to humans comparable adult weight of 70 kg earlier than a minipig. Moreover, a model of the present invention can mimick true obese weights of for example 100-150 kg. In the present invention it was found that the selection of the type of pig is an important factor in the clinical manifestation of diabetes mellitus type II or syndrome X. Diabetes mellitus type II or Syndrome X are caused by a combination of genetic and environmental factors as explained previously. Since diabetes mellitus type II and/or Syndrome X predominantly manifests itself at adolescent-age onwards, it is a significant advantage to possess an animal model which represents adolescents and adults in weight, size and built. The invention provides such an animal model.

By controlled variation of environmental factors the animal model of the present invention not only represents a patient suffering from Syndrome X. Analysis of the effects of variations in environmental factors can indicate (importance of) risk factors.

In a preferred embodiment the invention provides a method of the invention wherein said pig exhibits a plasma triglyceride level in a fasting phase higher than 0.8 mmol/L, preferably higher than 1.0 mmol/L. The invention further provides a method according to the invention wherein said plasma triglyceride level in a postprandial phase is higher than 1.0 mmol/L, preferably higher than 1.2 mmol/L. In a preferred embodiment the invention provides a method, wherein said pig shows elevated plasma non-esterified fatty acids (NEFA) concentrations in a postprandial phase compared to a normal, untreated, pig. Preferably the plasma NEFA concentrations are 5-fold elevated or more compared to a normal, untreated, pig.

Destruction of pancreatic β-cells can be achieved in various ways. Destruction is for example established by an attack of the immune-system. Alternatively destruction is caused by a surgical operation or by administration of any substance that will damage the pancreatic β-cells. Many of the available methods are capable of establishing partial destruction besides causing destruction of the entire entity of present pancreatic β-cells. For example, a part of the pancreas can be surgically removed. Several chemical substances can also cause partial destruction of the pancreas. In a preferred embodiment the invention provides a method, wherein said partial destruction of said pancreatic beta-cells is achieved by administration of a substance that is preferentially toxic to pancreatic β-cells. In one embodiment partially destructing pancreatic beta-cells involves at least partially diminishing insulin production by said pancreatic β-cells, while said pancreatic β-cells are not dysfunctional in all aspects. Hence according to this embodiment the pancreatic β-cells are not dysfunctional in all aspects as long as insulin production is at least in part impaired. Insulin production is for instance impaired by at least in part inhibiting expression and/or excretion of insulin, for example using siRNA.

A substance that preferentially destructs pancreatic β-cells is preferably a glucose-molecule coupled with a toxic component. Pancreatic β-cells possess receptors with high affinity for glucose-molecules and therefore they bind such a substance as it circulates in the blood. After binding of the substance to the pancreatic β-cells these cells can be destroyed by the toxic component. In a preferred embodiment said substance is streptozotocin (STZ) or a functional equivalent or derivative thereof. A functional equivalent or derivative of STZ is defined as a STZ compound which has been altered such that the pancreatic beta cells destroying properties of said compound are essentially the same in kind, not necessarily in amount. STZ is a substance that has little side-effects. Another preferred β-cell-toxic substance is for alloxan. Alloxan is a substance with well-known characteristics and is easy to obtain. A type-II like diabetes mellitus has been generated in rats by STZ administration (Blondel et al., 1990; Kergoat, Portha, 1985; Kruszynska, Home, 1988; Reed et al., 1999). This type-II like diabetes however does not resemble type II diabetes in a human being.

The type of diabetes induced by administration of a substance depends on the administration strategy. Administration route, speed of administration and release-formula are part of an administration strategy. In a highly preferred embodiment of the invention a substance capable of inducing diabetes mellitus type II or Syndrome X in a pig of the invention is administered intravenously. In a preferred embodiment of the invention said STZ or functional equivalent or derivative thereof is made available by slow infusion or a slow-release formula. Slow herein is defined as lasting at least 10 minutes, preferably 30 minutes. In a preferred embodiment of the invention said STZ or functional equivalent or derivative thereof is administered in a dosage in the range of 110 to 130 mg/kg. In a particularly preferred embodiment, the invention provides a method for selecting a pig wherein pigs are injected with a substance that is preferentially toxic to pancreatic β-cells and measuring fasting plasma glucose levels after these levels have stabilized (usually after one night) and selecting a pig with the desired fasting plasma glucose level. This administration and measurement is repeated when the fasting plasma glucose level in the measured pig is lower than desired. The dosage of said substance that is preferentially toxic to pancreatic β-cells that is given per administration can be adjusted as desired and is preferably between 5 and 20 mg/kg, more preferably between 7 and 15 mg/kg and particularly preferred about 10 mg/kg. In this way, particularly when using STZ, the model can be titrated exactly. Administration of the substance that is preferentially toxic to pancreatic β-cells in several divided doses is particularly preferred for developing a Syndrome X model. Administering divided doses coupled with repeated measurements of fasting plasma glucose levels allows optimal control over the level of fasting plasma glucose that is obtained in a method of the invention and therefore allows standardization of at least one parameter in the model even when using an outbred population. When administering divided doses, it is preferred to provide doses with intervals of at least one day. When administering divided doses it is not necessary to prevent peak levels of the toxic substance. In these cases intravenous injection is not a problem. In a preferred embodiment of the invention a Syndrome X model is generated by daily administration of about 10 mg/kg STZ or functional equivalent or derivative thereof until a desired fasting plasma glucose level is achieved.

In one embodiment pigs of the invention are challenged by environmental factors which contribute to the induction of type II diabetes mellitus and/or Syndrome X. Examples of environmental factors, which pigs of the invention are preferably exposed to are a specific diet and/or low physical activity. Exposure to low physical activity is for example established by restraining pigs in their motion, for instance by accommodating them in small cages. Said diet is preferably low in proteins and/or high in fat and/or sugars. In one embodiment of the invention, a pig of the invention is challenged by low physical activity and/ or a diet that is high in fat percentage and carbohydrates. Said high fat percentage preferably comprises a high cholesterol percentage. In a preferred embodiment of the invention, a pig of the invention is challenged by low physical activity and a diet which has a fat percentage of about 15%, a glucose, preferably sucrose, percentage of about 20% and a cholesterol percentage of about 1%. A pig of the invention that is challenged with low physical activity and/or with said diet comprising a high fat percentage and/or a high sugar and/or cholesterol content, is preferably used as a model for Syndrome X. Therefore, in a preferred embodiment a method of the invention further comprises challenging a pig of the invention with a diet which is high in fat and/or cholesterol and/or glucose and/or challenging said pig with low physical activity. In a further preferred embodiment of the invention a Syndrome X model is generated in a pig by daily administration of 10 mg/kg STZ or functional equivalent or derivative thereof to said pig until a desired fasting plasma glucose level is achieved and by challenging said pig with a diet which is high in fat and/or cholesterol and/or glucose and/or by challenging said pig with low physical activity.

Body weight is an important feature of the model of the present invention. Desired body weights can be achieved by providing the pigs with an appropriate diet. The diet can also be used to achieve a certain desired fat percentage in model pigs of the invention either prior to or subsequent to partially removing pancreatic beta cells from the body. In one embodiment the invention provides aforementioned method, wherein said pig has a percentage of fat of at least 15%, preferably of at least 25%. Wherein "has a percentage of fat" is defined as possessing a certain weight percentage of total body weight as fat cells. The invention further provides a pig defined by a weight of 70-150kg at 5-9 months, obtainable by a method according to the invention. The invention also provides a pig according to the invention, wherein said pig has a percentage of fat of at least 15%, preferably of at least 25%. Desired fat percentages can also be achieved by providing a breed of pigs that naturally converts diet in a higher percentage of fat. In a preferred embodiment the invention therefore provides a pig according to the invention, wherein said pig is a pig of a Pulawska breed or a pig of a Meishan breed. Pulawska and Meishan breeds are for example described by Valerie Porter in Pigs, a handbook to the breeds of the world (published by Helm Information, 1993). A breed society of a Pulawska pig can for instance be found in Warsaw, Poland, located at the Faculty of Animal Sciences, department of genetics and animal breeding. A Pulawska breed as well as a Meishan breed by nature has a larger fat percentage than a domestic pig. By providing a breed which has succeeded to incorporate this characteristic of a Pulawska or a Meishan breed, a model of the invention provides a model to represent obesity. In one embodiment the invention provides a method according to the invention, wherein said pigs have a genetic background for obesity, preferably central obesity. Central obesity is associated with both diabetes mellitus type II and Syndrome X. The strongest association is with Syndrome X as it appears to be one of its main predisposing factors. In a preferred embodiment, the breed used for developing a model pig of the invention is a Pulawska breed.

In research comprising the use of animal models a source of bias is sampling living animals. Sampling conscious animals almost always causes stress, stress introduces many undesired side-effects in the experiment, unless of course you would want to measure stress effects but then still the amount of stress induced would have to be controllable. One of the 'solutions' in order to prevent stress from occurring in an animal is to anaesthetize an animal. In that situation however, you do get other undesired side-effects, from the anaesthesia, instead. It is an advantage to be able to sample an animal without or with a minimum of stress. A sample derived from an animal without or with a minimum of stress is, for example, more reliable than a sample derived from an animal with a significant stress-response, because of the physiological changes that occur in the body of a stressed animal. For example blood-glucose levels often change considerably when a stress-response occurs. Hence, it is preferred to at least in part prevent a stress response in an animal when a sample is taken.

Another positive aspect of being able to sample an animal without or with a minimum of stress is a positive effect on an animals well-being. One embodiment of the invention sees to at least in part preventing stress by providing an animal equipped with a catheter by which means fluids can be sampled without causing stress or causing a minimum level of stress. In a long-lasting experiment being able to apply a permanent catheter saves time, money and stress. The invention provides a pig according to the invention, wherein said pig is equipped with at least one catheter in at least one body-compartment. A body-compartment is defined as any part of a body that is accessible for catheterisation. In a preferred embodiment of the invention a body-compartment comprises a blood vessel, a lymph vessel or a bowel. Said lymph vessel preferably is a Ductus Thoracicus, since this large vessel is easily equipped with a catheter. Said blood vessel preferably is a portal vein. In a preferred embodiment the invention provides a pig according to the invention, wherein said pig is equipped with at least one catheter in at least one body-compartment, wherein said catheter is permanent. Wherein permanent is defined as any time period allowing for multiple administrations and/or samplings. Permanent catheters are typically present over a period of days, typically more than several weeks. In a preferred embodiment the invention provides a pig of the invention equipped with a portal vein catheter.

According to the present invention it is possible to insert a portal vein catheter in a mammal via a splenic vein of said mammal. Said portal vein catheter is therefore preferably inserted in said pig via a splenic vein of said pig. A method for inserting a portal vein catheter in a mammal, comprising inserting said catheter via a splenic vein of said mammal, is therefore also herewith provided.

Glucose metabolism is an important feature in the development of diabetes mellitus type II and Syndrome X but also in many other disorders as it is a basic metabolic route in vertebrates. For example the brain has glucose as its main energy source. In one embodiment the invention provides the use of a pig according to the invention for manipulation of glucose metabolism. Manipulation herein is defined as an intervention which has an influence on glucose metabolism, it can for instance be a treatment. Blood sugar levels are kept within a normal healthy range by the hormone insulin. In a preferred embodiment the invention provides the use of a pig according to the invention for manipulation of glucose metabolism, wherein said glucose metabolism is insulin-mediated. The invention further provides the use of a pig according to the invention for manipulation of glucose metabolism, wherein said manipulation comprises manipulation of type II diabetes mellitus or Syndrome X.

The invention preferably provides use of a selected pig according to the invention for evaluating effects of a treatment on the manifestation of type II diabetes mellitus or syndrome X. In a preferred embodiment the invention provides use of a pig according to the invention, wherein said treatment comprises administration to said pig of nutrition elements or nutrition compounds. Effects of administration of nutrition elements and nutrition compounds to aforementioned animal model can be evaluated before and after induction of diabetes mellitus type II or syndrome X. The invention further provides a use of a pig according to the invention, wherein said treatment comprises administration to said pig of a pharmaceutical composition. Pharmaceutical substances that are available for the treatment of diabetes type II and can be evaluated for its effects in a pig of the invention are, for example, acetohexamide, glipizide, glyburide or tolazamide. In a preferred embodiment of the invention it provides the use of a selected pig according to the invention, wherein said pharmaceutical composition is metformin. Metformin is a medicine frequently used by human diabetics. In human studies, it is a normal phenomenon that the metformin treatment needs to be continued for several weeks before a stable effect of metformin on glucose homeostasis can be observed (Bailey, Turner, 1996; Cusi, DeFronzo, 1998). By contrast, rodent studies,(Reed et al., 1999; Cheng et al., 2001; Dutta et al., 2001; Leng et al., 2004; Pushparaj et al., 2001; Suzuki et al., 2002) reported an immediate (within days) effect of metformin at relatively high doses (100-500 mg/kg) on glucose homeostasis. The magnitude of improvement by metformin on glycemic control in pigs of the invention is comparable to that reported in human studies.

In a further preferred embodiment the invention provides a use of a selected pig according to the invention for evaluating effects of a treatment on the manifestation of type II diabetes mellitus or syndrome X, wherein said treatment comprises pancreatic beta-cell transplant. Symptoms of diabetes mellitus type II or syndrome X in aforementioned animal model can be evaluated before and after transplant of pancreatic beta-cells. In a highly preferred embodiment the invention therefore provides the use of a pig according to the invention for evaluating effects of a pancreatic beta-cell transplant.

The invention further provides a collection of outbred pigs selected by means of a method of the invention. The invention further provides a collection of pigs having a fasting plasma glucose level higher than 6 mmol/L, preferably 10 mmol/L or higher. Preferably said collection of pigs is of a Pulawska breed or a pig of a Meishan breed. Preferably of a Pulawska breed. In another preferred embodiment said collection of pigs is a cross-breed of a Yorkshire x Landrace breed. Preferably, at least one pig of said collection of pigs comprises a permanent catheter. Preferably each of pig of said collection of pigs comprises a catheter.

Diabetes type II and Syndrome X share some causal factors and have a few deregulations of endocrinal and metabolic mechanisms in common. Deregulations of endocrinal and metabolic mechanisms in general are a common cause of many diseases. An animal model adequately representing one of those diseases is an appropriate model for related relevant endocrinal and metabolic disorders. An animal model provided by the invention has an anatomical, physiological and metabolic resemblance to human which makes it suitable for the investigation of various deregulations of endocrinal and metabolic mechanisms. The invention provides the use of a pig according to the invention for manipulation of metabolic and/or endocrine disorders.

The invention is further illustrated by the following example. The example does not limit the scope of the invention in any way.

### Example

### Materials and Methods

### Animals and housing

Experimental protocols describing the management, surgical procedures, and animal care were reviewed and approved by the ASG-Lelystad Animal Care and Use Committee (Lelystad, The Netherlands).
Thirty-six crossbred pigs (Yorkshire x Landrace) of approximately 30 kg BW at surgery were used in this study. Two weeks before surgery the pigs were housed in metabolism cages (1.15 x 1.35 m) and adapted to the light/dark cycle and the feeding regimen. Lights were on and off at 05:00 and 22:00 h, respectively. Ambient room temperature was 20 °C.

### Feeding regimen and surgery

A commercial diet (5% crude fat, 16% crude protein, 41% starch and sugars, 20% non-starch polysaccharides, 6% ash and 12% water; Startbrok; Agrifirm, Meppel, The Netherlands) was fed twice daily, i.e. at 06:00 and 15:00 h at free access to water. Pigs were weighed twice weekly and meal size was adjusted to the weight of the pig. The nutritive value was equal to 2.5-fold maintenance requirements for ME (metabolizable energy) as established in a normal pig (CVB 2000). This corresponded with a feeding level of 1045 kJ ME/kg BW^{0.75} (metabolic weight of the pigs) per day sufficient for moderate growth in a normal pig.

The surgery was preceded with a 24-h period of fasting, and only water was offered. The day after surgery, the pigs were given 50% of the pre-surgically consumed food, and afterwards their preoperative food intake was established.

Pigs were anesthetized by intramuscular injection of 2 mg azaperone/kg BW (Stressnil; Janssen, Tilburg, The Netherlands) followed by an intravenous injection of 15 mg Nesdonal/kg BW (Rhone Merieux, Lyon, France). Pigs were intubated and general anesthesia was maintained by inhalation of 4% isoflurane in combination with O₂ and N₂O. Afterwards, pigs were equipped with two polyethylene catheters (Tygon, i.d. 1.02 mm, o.d. 1.78 mm, length 1 m; Norton, Akron, Ohio, USA) in the right carotid artery and the right external jugular vein according to a modified procedure (Koopmans et al., 2003). The catheters were inserted and advanced until the tip of the catheter reached the aorta (carotid artery catheter) or the antrum (jugular vein catheter). The catheters were fixed firmly at the place of insertion and were tunnelled subcutaneously to the back of the pig and exteriorized between the shoulder blades. The catheters were filled and sealed with physiological saline containing 50 IU heparin and 150.000 IU penicillin (Procpen; AUV, Cuijk, The Netherlands) per mL and kept in and protected by a back pack which was glued to the skin of the pig's back. During surgery the pig was given an intramuscular injection of antibiotic (300.000 IU procaine penicilline G, Depocilline, Mycofarm Nederland B.V., De Bilt, The Netherlands) and anodyne (50 mg flunixine, Finadyne, Schering-Plough N.V./S.A., Brussel, Belgium).

During the one week recovery period after surgery, the pigs were habituated to the blood sampling and infusion procedure. The carotid artery was used for blood sampling and the jugular vein catheter was used for the infusion of fluids. During the blood sampling procedure, the catheters were flushed and filled with physiological saline containing 5 IU heparin per mL. After the one week postsurgical recovery/habituation period, the pigs were assigned for a hyperinsulinemic clamp experiment or were treated with streptozotocin to induce diabetes.

### Induction of diabetes

STZ (Pharmacia & Upjohn Company, Kalamazoo, Michigan, USA) was dissolved in saline (1 g/10 mL) and administrated to pigs via the jugular vein by a continuous infusion over 30 minutes. STZ treatment (110, 130 or 150 mg/kg) was initiated 4-5 hours after the morning meal. During the first 3 days after STZ treatment, food was offered ad libitum in order to avoid hypoglycemia (Gäbel et al., 1985; Grussner et al., 1993). Over the following 4 days, the food was offered still ad libitum but now from 06:00 to 7:00 and from 15:00 to 16:00 h to evaluate the food intake capacity in relation to 24-h urinary glucose excretion of diabetic pigs. This 1) to be sure that diabetic pigs consume their meals at the iso-energetic level of 1045 kJ ME/kg BW^{0.75} per day and 2) to underline the importance of iso-energetic feeding when 24-h urinary glucose excretion is used as one of the parameters to quantify the efficacy of metformin in diabetic pigs. On day 8 post STZ treatment, pigs were fed on a restricted and iso-energetic level of 1045 kJ ME/kg BW^{0.75} per day throughout the study.

### Study protocols

In total, 36 pigs were used in 5 study protocols, comprising 58 experiments in the non-diabetic (normal) and/or diabetic state. Twenty-two pigs were used in 2 study protocols. A 1 week time interval was imposed between the 2 study protocols when a pig was re-used.
Protocol 1: fasting hepatic glucose production and whole body glucose uptake were studied with 6,6-²H-glucose infusion in 5 normal and 5 STZ (130 mg/kg) diabetic pigs.
Protocol 2: insulin-mediated hepatic glucose production and whole body glucose uptake were studied with the hyperinsulinemic (1 or 2 mU/kg.min) euglycemic clamp technique in combination with 6,6-²H-glucose infusion in 6 normal and 7 STZ (130 mg/kg) diabetic pigs.
Protocol 3: insulin-mediated whole body glucose disposal was studied with the hyperinsulinemic (2 or 8 mU/kg.min) euglycemic clamp technique in 6 normal and 6 STZ (150 mg/kg) diabetic pigs.
Protocol 4: insulin mediated whole body glucose disposal under influence of acute mass action of plasma glucose was studied with the hyperinsulinemic (2 mU/kg.min), euglycemic (6 mmol/L) and hyperglycemic (22 mmol/L) clamp technique in 4 STZ (110 mg/kg) diabetic pigs.
Protocol 5: insulin mediated whole body glucose disposal, after a 2 week placebo or metformin (twice 1.5 g per day) oral treatment period under conditions of iso-energetic feeding, was studied with the hyperinsulinemic (2 mU/kg.min) euglycemic clamp technique in 14 STZ (130 mg/kg) diabetic pigs.

### Infusates

Insulin (Actrapid MC, porcine monocomponent, Novo, Copenhagen, Denmark), 6,6-²H-glucose (Cambridge Isotope Laboratories, Inc, MA, USA) and D-glucose (Merck, Darmstadt, Germany) were prepared as sterile solutions and passed through a 0.22 µm Millipore filter into sterile containers before use. Insulin was diluted in a saline solution containing 3% pig plasma in order to avoid sticking of insulin to the plastic containers and tubings. 6,6-²H-glucose was dissolved in a saline solution and D-glucose was dissolved in aqua dest.

### Fasting glucose rate of appearance

After overnight fasting, a 6,6-²H-glucose solution was administered as a prime (72 mg)-continuous (1.2 mg/min) infusion for 3 hours in normal pigs. In diabetic pigs, 6,6-²H-glucose was given as an adjusted prime (216 mg)-continuous (3.6 mg/min) infusion for 3 hours (Koopmans et al., 1991). Isotopic steady state conditions were achieved within 90 minutes after initiation of the tracer infusion and steady state calculations were carried out during the last hour of the tracer infusion (t= 120, 135, 150, 165 and 180 min).

### Hyperinsulinemic euglycemia and glucose rate of appearance

After overnight fasting, at t= 0 min, insulin was administered as a prime (17 or 34 mU/kg)-continuous (1 or 2 mU/kg.min) infusion for 3 hours in normal pigs. Simultaneously, a variable infusion of a 33% D-glucose solution was started and adjusted every 10 minutes to maintain the plasma glucose concentration at euglycemic levels and a 6,6-²H-glucose solution was given as a prime (216 mg)-continuous (3.6 mg/min). Steady state conditions were achieved within 90 minutes after initiation of the hyperinsulinemic clamp and steady state calculations were carried out during the last hour of the clamp (t= 120, 135, 150, 165 and 180 min). In hyperglycemic diabetic pigs, insulin was given as a prime (34 or 136 mU/kg)-continuous (2 or 8 mU/kg.min) infusion for 5 hours. After reaching euglycemic levels, a variable infusion of a 33% D-glucose solution was started and adjusted every 10 minutes to maintain the plasma glucose concentration at euglycemia. At t=2 hours, 6,6-²H-glucose was administered as a prime (216 mg)-continuous (3.6 mg/min) infusion for the last 3 hours of the clamp in diabetic pigs. Steady state conditions were achieved during the last hour of the clamp and steady state calculations were carried out at (t= 240, 255, 270, 285 and 300 min).

### Hyperinsulinemic hyperglycemia

Hyperinsulinemic hyperglycemic clamp experiments were carried out in diabetic pigs only. After overnight fasting, insulin was given as a prime (34 mU/kg)-continuous (2 mU/kg.min) infusion for 5 hours. Simultaneously, a variable infusion of a 33% D-glucose solution was started and adjusted every 10 minutes to maintain the plasma glucose concentration at hyperglycemia. Steady state calculations were carried out during the last hour of the clamp (t=240, 255, 270, 285 and 300 min).

### Fasting and postprandial blood samples

Fasting blood samples were taken before the start of hyperinsulinemic clamp experiments and postprandial blood samples were taken 2-3 hours after a morning meal in the week following a clamp study.

### Plasma, urine and infusate analyses

Blood samples for determination of 6,6-²H-glucose enrichment, glucose and insulin were collected in heparinized tubes (150 USP. U. Lithium Heparin, 10 mL Venoject, Terumo, Leuven, Belgium) and immediately chilled at 0° C on ice, and centrifuged at 4° C for 10 minutes at 3000 rpm. Plasma was stored at - 20° C in 4 aliquots of 0.5 mL for further analyses. Glucose was extracted from the plasma with methanol, followed by derivatising with hydroxylamine and acetic anhydrid as described previously (Reinauer et al., 1990). The aldonitrile pentaacetate derivative was extracted in methylene chloride and evaporated to dryness in a stream of nitrogen. The extract was reconstituted with ethylacetate and injected into a gas chromatograph/mass spectrometer system (HP 6890 series GC system and 5973 Mass Selective Detector). Separation was achieved on a J&W scientific DB 17 capillary column (30 m x 0.25 mm x 0.25 µm). Selected ion monitoring, data aquisition and quantitative calculations were performed using the HP Chemstation software. Glucose concentration was determined using the internal standard method (xylose was added as internal standard). The internal standard was monitored at m/z 145. Glucose was monitored at m/z 187 for glucose and m/z 189 for d₂-glucose. Glucose enrichments were calculated by dividing the area of the m/z 189 peak and the m/z 187 peak and correcting for natural enrichments. Isotopic enrichment was calculated as tracer-to-tracee ratio after subtracting the isotopic enrichment of a background plasma sample. An aliquot of the 6,6-²H-glucose infusate was analysed for the isotope concentration to calculate the actual infusion rate for each infusion experiment.

Plasma insulin concentration was measured using a Delfia assay (test kit No B080-1101 by Perkin Elmer Life sciences, trust by Wallac Oy, Turku, Finland). This specific pig insulin assay was validated using pig insulin standards. Plasma and urine glucose concentrations were analysed enzymically on an autoanalyzer of Radiometer (ABL and AML, Copenhagen, Denmark). Urine was collected in buckets containing 0.5 grams Halamid-d (sodium-p-toluenesulfonchloramide, Akzo Nobel Chemicals, Amersfoort, The Netherlands) to prevent microbial breakdown of glucose. Urine volume was registered over a given interval in order to calculate the rate of urinary glucose excretion. Urine samples (0.5 mL) were stored at -20° C for later glucose analyses. Ketones (acetoacetic acid) were determined in fresh urine by a reagent strip test (Ketostix, Bayer Diagnostics, Mijdrecht, The Netherlands).

Blood samples for determination of triglycerides and non-esterified fatty acids (NEFA) were collected in tubes containing EDTA (0.47 mol/L EDTA, 10 mL Venoject, Terumo, Leuven, Belgium) and immediately chilled at 0° C on ice, and centrifuged at 4° C for 10 minutes at 3000 rpm. Plasma was stored at - 20° C in 4 aliquots of 0.5 mL for further analyses.

Concentrations of triglycerides, cholesterol and NEFA were measured using commercially available test kits (Boehringer Mannheim, Mannheim Germany; Human, Wiesbaden, Germany; and Wako Chemicals, Neuss, Germany, respectively).

### Calculations

### 6,6-²H-glucose kinetics:

Fasting and insulin-mediated rate of appearance (Ra) of glucose was calculated by dividing the 6,6-²H-glucose infusion rate (mg/min) by the plasma 6,6-²H-glucose enrichment (mg/mg %). Insulin-mediated hepatic glucose production was calculated as measured Ra glucose minus exogenous glucose infusion rate. Under hyperglycemic conditions, the rate of disappearance (Rd) of glucose, or whole body glucose uptake was corrected for urinary glucose excretion. This implies that whole body glucose uptake was calculated as measured Ra glucose minus urinary glucose excretion.

### Statistical methods and analyses

Multiple factorial comparisons were submitted to analyses of variance (ANOVA), if applicable ANOVA for repeated measurements, and followed by the unpaired Student's t-test as a post-hoc analysis. Statistical significance between averages from 2 data sets (as between subject comparison) were performed by the unpaired Student's t-test. Statistical significance between averages from 2 data sets (as within subject comparison) were performed by the paired Student's t-test. The results are expressed as means±SEM .The criterion of significance was set at p<0.05.

### Results

### Induction of diabetes

One week post STZ treatment, 2 of 6 pigs receiving a dose of 110 mg/kg BW and 3 of 23 pigs receiving a dose of 130 mg/kg BW had overnight fasting plasma glucose concentrations <10 mmol/L and were excluded from the study. All 6 pigs treated with a STZ dose of 150 mg/kg BW showed overnight fasting plasma glucose concentrations >20 mmol/L.

### Food intake and urinary glucose excretion

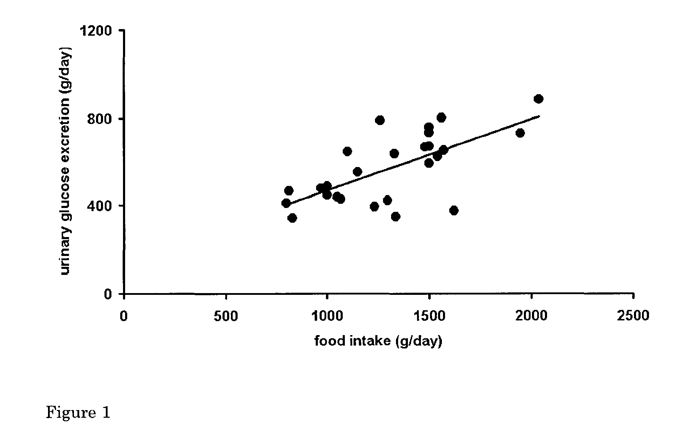

After inducing diabetes with STZ (4-7 days), food was offered to pigs ad libitum from 06:00 to 7:00 and from 15:00 to 16:00 h to evaluate the rate of food intake in relation to 24-h urinary glucose excretion of diabetic pigs (Figure 1). Ad libitum food intake ranged from 800 to 2036 g per day and 24-h urinary glucose excretion ranged from 341 to 886 g per day. From linear regression analysis, the equation was calculated to be y = 0.33x + 141 (R=0.67) with a highly significant (p<0.0002) correlation between both parameters. The slope of the line predicted that each g of feed intake (i.e. 0.41 gram starch and sugars) leads to 0.33 g of urinary glucose excretion. The intercept was equal to the fasting 24-h urinary glucose excretion (141 g per day). Average body weight of all diabetic pigs was 37.3±1.2 kg, and thus their predicted fasting urinary glucose excretion was equal to 2.6 mg/kg.min. To measure a factual rate of fasting urinary glucose excretion, diabetic pigs were fasted overnight and afterwards urine was collected for 8 h. It appeared that the factual fasting urinary glucose excretion was similar to the predicted value and amounted 2.3±0.2 mg/kg.min.

In urine of diabetic pigs (STZ dose of 110 and 130 mg/kg) no or trace (0.5 mmol/L) amounts of ketones were detected, whereas STZ at a dose of 150 mg/kg induced intermediate to moderately high levels of ketones in urine (1.5-4 mmol/L).

### Glucose metabolism and insulinemia.

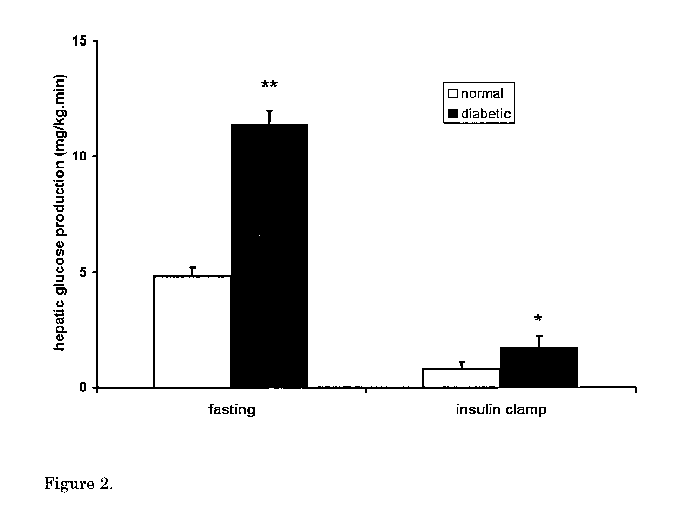

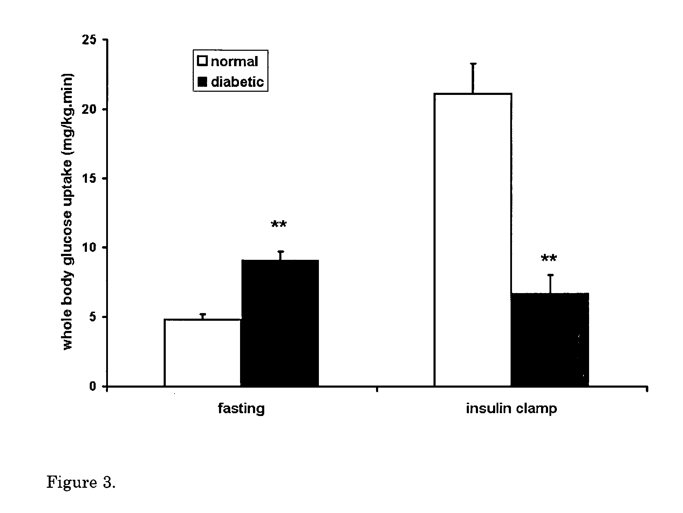

Glucose metabolism in normal and diabetic (STZ doses of 110, 130 and 150 mg/kg) pigs after overnight fasting and after insulin infusion at the rate of 1, 2 or 8 mU/kg.min is presented in Tables 1 and 2 and Figures 2 and 3. Overnight fasting plasma glucose concentrations were on average 4-fold greater in the 3 diabetic pig groups compared to normal pigs. Overnight fasting plasma insulin concentrations however, were comparable and did not differ significantly among normal and diabetic pigs. Diabetic pigs treated with STZ (110 and 130 mg/kg) had a wide range (4-51 and 1-38 mU/L) in fasting plasma insulin concentrations, which on average were 2-fold higher than in normal and diabetic (150 mg STZ/kg) pigs which showed a narrow range (2-10 and 0-6 mU/L) in fasting plasma insulin concentrations (Tables 1 and 2). Fasting Ra glucose was approximately 2-fold greater in diabetic (130 mg STZ/kg) pigs compared to normal pigs. Insulin inhibited hepatic glucose production was 2-fold greater and insulin stimulated whole body glucose uptake was 3-fold lower in diabetic (130 mg STZ/kg) versus normal pigs (Figures 2 and 3).

Diabetic pigs treated with STZ (150 mg/kg) required an insulin infusion rate of 8 mU/kg.min during the hyperinsulinemic euglycemic clamp to reach near euglycemic levels within the time frame (5 h) of the clamp study. The steady state glucose infusion rate was ~8-fold lower compared to the average glucose infusion rates achieved in normal pigs (Table 1). For diabetic pigs treated with STZ (110-130 mg/kg), an insulin infusion rate of 2 mU/kg.min proved to be sufficient to reach near euglycemic levels within the time frame (5 h) of the clamp study. The steady state glucose infusion rates were 5- to 6-fold lower compared to the average glucose infusion rates achieved in normal pigs (Table 1).

To study acute mass action of plasma glucose on insulin stimulated whole body glucose disposal, diabetic pigs (STZ 110 mg/kg) were clamped both at euglycemic and hyperglycaemic levels (Table 2). Insulin-mediated whole body glucose disposal was increased by 16% (5.9 vs 5.1 mg/kg.min, p<0.05) at hyperglycemia compared to euglycemia.

### Lipidemia

Plasma concentrations of triglycerides, cholesterol and NEFA in normal and diabetic (STZ 130 mg/kg) pigs are shown in Table 3. Fasting plasma triglyceride concentrations were found to be 5-fold elevated in diabetic pigs compared to normal pigs whereas fasting cholesterol and NEFA concentrations did not differ between both groups. In the postprandial phase of diabetic pigs, plasma triglycerides, cholesterol and NEFA concentrations were elevated (4-fold, 1.3-fold and 7-fold, respectively) compared to normal pigs.

### Metformin treatment

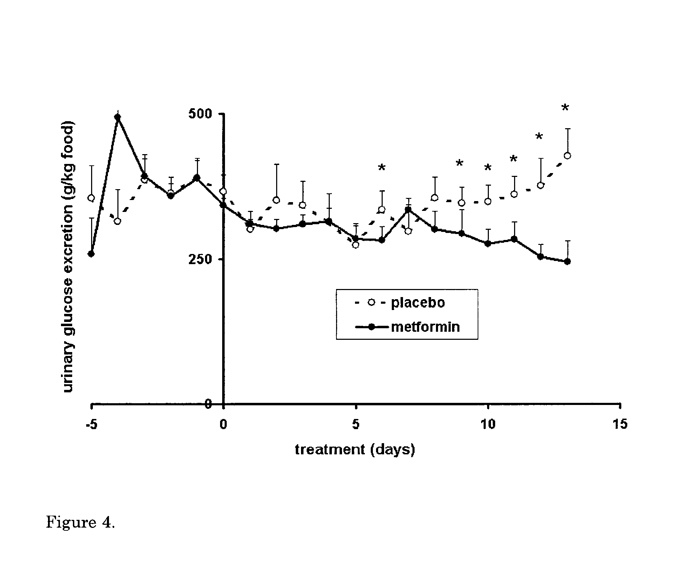

Postprandial plasma glucose and insulin concentrations at the beginning and the end of the 2-week placebo versus metformin treatment of diabetic pigs are shown in Table 4. Initial plasma glucose and insulin concentrations were similar among the pigs, whereas after the treatment period of 14 days with metformin, plasma glucose concentrations declined by 30% compared to placebo treated pigs at similar plasma insulin concentrations. Metformin treated diabetic pigs grew faster (+4.4±1.1 kg, p<0.01) than placebo treated diabetic pigs (+0.7±1.2 kg, p=NS).
Daily urinary glucose excretion (g/kg food) in diabetic pigs after metformin versus placebo treatment is depicted in Figure 4. Initial urinary glucose excretion was similar among both groups of pigs, but after one week of metformin treatment, urinary glucose excretion became significantly (p<0.05) less (20-40%) in the metformin group compared to the placebo group.

Basal plasma glucose concentrations were significantly (p<0.05) reduced at comparable basal plasma insulin concentrations in metformin treated pigs compared to placebo treated pigs (Table 5). Under similar insulin stimulation (3-4 fold over fasting insulin concentrations), whole body glucose disposal was found to be 1.6 fold greater in the metformin group compared to the placebo group with diabetes (9.4 vs 5.8 mg/kg.min).

This study demonstrates that the chemical induction of pancreatic beta-cell dysfunction in domestic pigs leads to insulin-resistant diabetes mellitus. We found that a STZ dose of 110 mg/kg did not lead to fasting hyperglycemia (<10 mmol/L) in 2 of 6 pigs, whereas a dose of 150 mg/kg resulted in a clear hyperglycemic, hypoinsulinemic, and ketotic diabetic state in all pigs. The latter implies that their diabetic state mimicked type 1 diabetes mellitus. A STZ dose of 130 mg/kg resulted in fasting hyperglycemia (>10 mmol/L) in 20 of 23 pigs with concomitant fasting normoinsulinemia. These diabetic pigs were non-ketotic, moderately dyslipidemic, developed severe insulin resistance for glucose metabolism, maintained growth without insulin therapy and resembled therefore type 2 diabetes mellitus.

From a quantitative point of view, the insulin resistance in pigs was mainly present at the level of whole body glucose uptake and to a lesser extent at the level of hepatic glucose production. This is consistent with human type 2 diabetes mellitus where peripheral (extrahepatic) tissues are the primary site of insulin resistance (DeFronzo et al., 1992; DeFronzo, 1987). Insulin resistance for glucose metabolism in pigs appeared within one week after STZ treatment and remained stable during the following 2 weeks of the study, which is useful when chronic mechanisms of pharma, nutraceuticals or functional foods on insulin resistance are investigated. In addition, the severity of insulin resistance in diabetic pigs was relatively independent on acute changes in plasma glucose concentrations since we have shown that insulin-mediated glucose metabolism is only 16% increased at hyperglycemia compared to euglycemia. This is in agreement with previous studies (Mandarino et al., 1996; Bevilacqua et al., 1985; Koopmans et al., 1992) which have shown that insulin resistance at euglycemia is only partially compensated at hyperglycemia. In STZ diabetic rats, a 30-50% reduction in insulin action was reported, (Koopmans et al., 1991; Blondel et al., 1990; Kruszynska, Home, 1988; Koopmans et al., 1992; Nishimura et al., 1989) but some rat studies could not demonstrate a defect in insulin action (Kergoat, Portha, 1985; Dallaglio et al., 1985). Studies in STZ diabetic dogs reported a 50% reduction,(Bevilacqua et al., 1985) or no reduction,(Tobin, Finegood, 1993) in insulin action. Compared to rodent and dog studies, insulin resistance in STZ diabetic pigs is reproducible (all 31 STZ diabetic pigs developed insulin resistance) and severe as manifested by a ~75% reduction in insulin action.

Fasting plasma insulin concentrations have invariably been found to be normal or increased in human type 2 diabetes mellitus. Even in studies where normal fasting plasma insulin levels have been reported, they uniformly have been in the high normal range.(DeFtonzo et al., 1992). We documented that mean fasting plasma insulin concentrations were similar in 130 mg/kg STZ diabetic pigs (mean 9, range 1-38 mU/L) compared to normal pigs (mean 5, range 2-10 mU/L). Normal fasting plasma insulin concentrations have been observed before in alloxan diabetic minipigs,(Kjems et al., 2001) but the studies with STZ diabetic (mini)pigs showed fasting hypoinsulinemia (Gäbel et al., 1985; Grussner et al., 1993; Ramsay, White, 2000; Marshall et al., 1980; Canavan et al., 1997; Larsen et al., 2002). As discussed previously,(Larsen et al., 2002) the discrepancy in fasting plasma insulin concentrations may be explained by different strain, age and gender but also by different β-cell-toxic compound (alloxan or STZ) and the way of STZ administration. We have infused STZ over a 30 min period whereas other studies injected STZ. We observed postprandial hypoinsulinemia (close to the detection limit of 1 mU/L) 2-3 h after feeding. The reason for this phenomenon is not clear but the acute prandial stimulation of insulin secretion superimposed on the chronic stimulation by hyperglycemia, may temporarily exhaust the insulin secretory capacity of the remaining β ―cells, 2-3 h postprandially.

Dyslipidemia in diabetic pigs was most manifested by elevated plasma triglycerides in the fasting (1.0 mmol/L) and postprandial (1.2 mmol/L) phases compared to normal pigs (0.2 and 0.3 mmol/L, respectively). Plasma cholesterol and NEFA concentrations were elevated in the postprandial phase only. The difference in fasting and postprandial dyslipidemia in diabetic pigs may have been caused by the differences in plasma insulin concentrations. Insulin is known to be a strong regulator of lipid metabolism,(Koopmans et al., 1996; Koopmans et al., 1999; Koopmans et al., 1998; Newsholm, Leech, 1989) and the diabetic pigs in our study had fasting normoinsulinemia but postprandial hypoinsulinemia. Despite dyslipidemic status is theoretically defined when the levels of plasma triglycerides are greater than 1.7 mmol/L,(Howard, Howard, 1994; National Cholesterol Education Program, 2001) it is clear that STZ diabetic pigs, fed a low fat diet, reveal an endogenous drive towards elevated plasma triglyceride concentrations compared to normal pigs. Thus, it may be expected that these pigs when fed a high fat, high cholesterol diet will develop a clear cut dyslipidemia (Gerrity et al., 2001).

### Metformin

We found that metformin treatment of diabetic pigs resulted in a 24% reduction in fasting plasma glucose concentrations, a 30% reduction in postprandial plasma glucose concentrations, a 20-40% reduction in 24-h urinary glucose excretion, and a 60% increase in insulin stimulated whole body glucose disposal, as compared to placebo treated diabetic pigs.

The magnitude of improvement by metformin on glycemic control in diabetic pigs is comparable to that reported in human studies (Bailey, Turner, 1996; Cusi, DeFronzo, 1998). The effect of metformin in pigs progressed over a 2 week time period and the reduction in 24 h urinary glucose excretion didn't reach steady state yet in diabetic pigs (Figure 2). In human studies, it is a normal phenomenon that the metformin treatment needs to be continued for several weeks before a stable effect of metformin on glucose homeostasis can be observed (Bailey, Turner, 1996; Cusi, DeFronzo, 1998). By contrast, rodent studies,(Reed et al., 1999; Cheng et al., 2001; Dutta et al., 2001; Leng et al., 2004; Pushparaj et al., 2001; Suzuki et al., 2002) reported an immediate (within days) effect of metformin at relatively high doses (100-500 mg/kg) on glucose homeostasis. These studies were of pharmacological use while our pig study was designed to serve a more physiological approach, using a dose of 3g metformin per day (~75 mg/kg).

### Iso-energetic feeding

STZ diabetic pigs become hyperphagic 1-2 weeks after STZ treatment (data not shown) which is in agreement with previous observations in rats (Koopmans et al., 1991; Koopmans et al., 1992). STZ treatment induces postprandial hypoinsulinemia which may induce, in combination with hypoleptinemia, (Ramsay, White, 2000) an increase in food intake (Koopmans et al., 1998; Schwartz et al., 1992). To exclude an effect of food intake on insulin sensitivity and glucose homeostasis, diabetic pigs were fed at the iso-energetic level of 1045 kJ/kg BW^{0.75} per day. This restricted feeding level proved sufficient for maintaining a constant body weight in diabetic pigs.

We found a significant correlation between daily ad libitum feed intake and 24-h urinary glucose excretion in diabetic pigs (Figure 1). This indicates that daily changes in food consumption (independent of major changes in body weight and body composition) strongly affect glucose homeostasis in diabetic pigs and underlines the necessity of iso-energetic feeding when studying anti-diabetic drug therapy. Indeed, it is well known that energy intake restriction can improve glucose homeostasis and tissue sensitivity to insulin (DeFronzo, Ferrannini, 1991). Part of the anti-diabetic effect of metformin may therefore stem from its ability to reduce food intake,(Lee, Morley, 1998; Paolisso et al., 1998) and in most studies food intake has not strictly been controlled (Reed et al., 1999; Cheng et al., 2001; Leng et al., 2004; Suzuki et al., 2002). Our study quantified the anti-diabetic effects of metformin at constant food intake.

**TABLE 1: Fasting and insulin-stimulated glucose metabolism in normal and diabetic pigs ¹⁾.**

| Pigs | Normal | Normal | Diabetic | Diabetic |
|---|---|---|---|---|
| Streptozotocin dose (mg/kg) | 0 | 0 | 130 | 150 |
| Insulin clamp (mU/kg.min) | 1 | 2 | 2 | 8 |
| 6,6-²H-glucose infusion | no | yes | yes | no |
| Fasting plasma glucose (mmol/L) | 5.3±0.2 | 4.8±0.2 | 21.7±1.1** | 22.0±0.8 |
| Fasting plasma insulin (mU/L) | 5±1 | 4±1 | 9±7 | 3±1 |
| | range (2-10) | | range (1-38) | |
| Time to reach euglycemia in diabetic pigs (min) | NA | NA | 145±22 | 243±21 |
| Steady state clamp plasma glucose (mmol/L) | 5.1±0.1 | 4.7±0.2 | 6.1±0.8 | 7.5±0.9 |
| Steady state clamp plasma insulin (mU/L) | 24±5 | 51±6 | 45±5** | 228±36 |
| Steady state clamp glucose infusion rate (mg/kg.min) | 20.3±2.1 | 28.1±1.4 | 5.0±1.4** | 3.2±1.0 |
| Body weight (kg) | 40.5±1.6 | 39.4±2.0 | 41.3±2.8 | 39.7±2.7 |

| | | | | |
|---|---|---|---|---|
| ¹⁾ Determined before and during hyperinsulinemic (insulin infusion rates of 1,2 or 8 mU/kg.min) euglycemic clamp studies with or without 6,6-²H-glucose infusion. Tracer derived results are depicted in Figures 2 and 3. NA=not applicable. **p<0.01 compared to normal pigs, 1 mU/kg.min clamp. | | | | |

**TABLE 2: Hyperinsulinemic (insulin infusion rate of 2 mU/kg.min) clamp studies at euglycemia or hyperglycemia in diabetic (STZ 110 mg/kg) pigs.**

| Clamp | euglycemia | hyperglycemia |
|---|---|---|
| Fasting plasma glucose (mmol/L) | 22.3±2.1 | 23.0±2.4 |
| Fasting plasma insulin (mU/L) | 17±13 range (4-51) | 12±5 range (3-21) |
| Steady state clamp plasma glucose (mmol/L) | 6.0±0.1 | 22.4±2.6 |
| Time to reach euglycemia in diabetic pigs (min) | 139±23 | NA |
| Steady state clamp plasma insulin (mU/L) | 54±16 | 62±19 |
| Steady state clamp glucose infusion rate (mg/kg.min) | 5.1±1.0 | 8.2±0.8*¹⁾ |
| Body weight (kg) | 46.9±1.7 | 47.7±3.5 |

| | | |
|---|---|---|
| ¹⁾Fasting urinary glucose excretion was estimated to be 2.3 mg/kg.min. Urinary glucose excretion was subtracted from the glucose infusion rate to obtain a measure of insulin-mediated whole body glucose disposal, being 5.9±0.8 mg/kg.min. NA=not applicable. *p<0.05. | | |

**TABLE 3: Plasma lipid profiles in the fasting and post-prandial state in normal and diabetic (STZ 130 mg/kg) pigs.**

| Pigs | Normal | Diabetic |
|---|---|---|
| Fasting plasma triglycerides (mmol/L) | 0.2±0.1 | 1.0±0.3** |
| Fasting plasma cholesterol (mmol/L) | 1.8±0.1 | 2.1±0.2 |
| Fasting plasma NEFA (mmol/L) | 1.0±0.3 | 0.9±0.3 |
| Postprandial plasma triglycerides (mmol/L) | 0.3±0.1 | 1.2±0.3** |
| Postprandial plasma cholesterol (mmol/L) | 1.7±0.1 | 2.3±0.2* |
| Postprandial plasma NEFA (mmol/L) | 0.2±0.1 | 1.5±0.5** |

| | | |
|---|---|---|
| *p<0.05, **p<0.01. | | |

**TABLE 4: Body weights and post-prandial plasma glucose and insulin concentrations of diabetic pigs, before and after placebo or metformin treatment over a period of 2 weeks.**

| Diabetic pigs | Placebo treated | Metformin treated |
|---|---|---|
| Body weight before treatment (kg) | 34.5±1.2 | 36.3±1.0 |
| Postprandial plasma glucose, before treatment (mmol/L) | 25.9±0.8 | 31.0±5.8 |
| Postprandial plasma insulin before treatment (mU/L) | 1±0 | 1±0 |
| Body weight at end of treatment (kg) | 35.2±2.4 | 40.7±1.8 |
| Postprandial plasma glucose, at end of treatment (mmol/L) | 35.5±4.9 | 24.9±2.2* |
| Postprandial plasma insulin, at end of treatment (mU/L) | 2±1 | 3±1 |

| | | |
|---|---|---|
| *p<0.05 | | |

**TABLE 5: Hyperinsulinemic (insulin infusion rate of 2 mU/kg.min) euglycemic clamps in diabetic pigs after placebo or metformin treatment over a period of 2 weeks.**

| Diabetic pigs | Placebo treated | Metformin treated |
|---|---|---|
| Fasting plasma glucose (mmol/L) | 19.4±0.6 | 14.7±1.5* |
| Fasting plasma insulin (mU/L) | 14±9 range (1-55) | 11±6 range (3-37) |
| Steady state clamp plasma glucose (mmol/L) | 6.1±0.9 | 5.3±0.3 |
| Time to reach euglycemia in diabetic pigs (min) | 175±36 | 105±28 |
| Steady state clamp plasma insulin (mU/L) | 48±6 | 42±7 |
| Steady state clamp glucose infusion rate (mg/kg.min) | 5.8±1.7 | 9.4±2.2* |

| | | |
|---|---|---|
| *p<0.05 | | |

### References

1. Gäbel H, Bitter-Suermann H, Henriksson C, et al: Streptozotocin diabetes in juvenile pigs. Evaluation of an experimental model. Horm Metab Res 17:275-280, 1985.
2. Grussner R, Nakhleh R, Grussner A, et al: Streptozotocin-induced diabetes mellitus in pigs. Horm Metab Res 25:199-203, 1993.
3. Ramsay TG, White ME: Insulin regulation of leptin expression in streptozotocin diabetic pigs. J Anim Sci 78:1497-1503, 2000.
4. Gerrity RG, Natarajan R, Nadler JL, et al: Diabetes-induced accelerated atherosclerosis in swine. Diabetes 50:1654-1665, 2001.
5. Porter P: Pigs, a handbook to the breeds of the world. Helm Information, 1993.
6. DeFronzo RA, Bonadonna RC, Ferrannini E: Pathogenesis of NIDDM. A balanced overview. Diabetes Care 15(3):318-368, 1992.
7. Mandarino LJ, Consoli A, Jain A, et al: Interaction of carbohydrate and fat fuels in human skeletal muscle: impact of obesity and NIDDM. Am J Physiol 270:E463-E470, 1996.
8. Bevilacqua S, Barrett EJ, Smith D, et al: Hepatic and peripheral insulin resistance following streptozotocin-induced insulin deficiency in the dog. Metabolism 34(9):817-825, 1985.
9. Koopmans SJ, de Boer SF, Sips HCM, et al: Whole body and hepatic insulin action in normal, starved, and diabetic rats. Am J Physiol 260:E825-E832, 1991.
10. Marshall M, Oberhofer H, Staubesand J: Early Micro- and Macro-Angiopathy in the Streptozotocin Diabetic Minipig. Res Exp Med (Berl) 177:145-158, 1980.
11. Canavan JP, Flecknell PA, New JP, et al: The effect of portal and peripheral insulin delivery on carbohydrate and lipid metabolism in a miniature pig model of human IDDM. Diabetologia 40:1125-1134, 1997.
12. Koopmans SJ, Sips HCM, Krans HMJ, et al: Pulsatile intravenous insulin replacement in streptozotocin-diabetic rats is more efficient than continuous delivery: effects on glycaemic control, insulin-mediated glucose metabolism and lipolysis. Diabetologia 39:391-400, 1996.
13. Blondel O, Bailbe D, Portha B: Insulin resistance in rats with non-insulin dependent diabetes induced by neonatal (5 days) streptozotocin: evidence for reversal following phlorizin treatment. Metabolism 39:787-793, 1990.
14. Kergoat M, Portha B: In vivo hepatic and peripheral insulin sensitivity in rats with non-insulin-dependent diabetes induced by streptozotocin. Diabetes 34:1120-1126, 1985.
15. Kruszynska YT, Home PD: Liver and muscle insulin sensitivity, glycogen concentration and glycogen synthase activity in a rat model of non-insulin dependent diabetes. Diabetologia 31:304-309, 1988.
16. Reed MJ, Meszaros K, Entes LJ, et al: Effect of masoprocol on carbohydrate and lipid metabolism in a rat model of type II diabetes. Diabetologia 42:102-106, 1999.
17. Marshall M: Induction of Chronic Diabetes by Streptozotocin in the miniature pig. Res Exp Med (Berl) 175:187-196, 1979.
18. Bailey CJ, Turner RC: Metformin. N Engl J Med 334:574-579, 1996.
19. Cusi K, DeFronzo RA: Metformin: a review of its metabolic effects. Diabetes Rev 6:89-131, 1998.
20. CVB 2000: Centraal Veevoederbureau. Dutch Norms for Livestock Feeding and Nutritive Values for Feedstuffs, 24th Ed CVB Press, Lelystad, The Netherlands.
21. Koopmans SJ, Mroz Z, Dekker R, et al: Insulin-stimulated net utilisation of plasma glucose and amino acids in growing pigs. In: Progress in research on energy and protein metabolism. EAAP 109:197-200, 2003.
22. Reinauer H, Gries FA, Hubinger A, et al: Determination of glucose-turnover and glucose-oxidation rates in man with stable isotope tracers. J Clin Chem Clin Biochem 28(8):505-511, 1990.
23. DeFronzo RA: The triumvirate: beta-cell, muscle, liver. A collusion responsible for NIDDM. Diabetes 37:667-687, 1987.
24. Koopmans SJ, Maassen JA, Radder JK, et al: In vivo insulin responsiveness for glucose uptake at eu- and hyperglycemic levels in normal and diabetic rats. Biochem Biophys Acta 115:230-238, 1992.
25. Nishimura H, Kuzuya H, Okamoto M, et al: Postreceptor defect in insulin action in streptozotocin-induced diabetic rats. Am J Physiol 256:E624-E630,1989.
26. Dallaglio E, Chang H, Hollenbeck CB, et al: In vivo and in vitro resistance to maximal insulin-stimulated glucose disposal in insulin deficiency. Am J Physiol 249:E321-E316, 1985.
27. Tobin BL, Finegood DT: Reduced insulin secretion by repeated low doses of STZ impairs glucose effectiveness but does not induce insulin resistance in dogs. Diabetes 42:474-483, 1993.
28. Kjems LL, Kirby BM, Welsh EM, et al: Decrease in β-cell mass leads to impaired pulsatile insulin secretion, reduced postprandial hepatic insulin clearance, and relative hyperglucagonemia in the minipig. Diabetes 50:2001-2012, 2001.
29. Larsen MO, Wilken M, Gotfredsen CF, et al: Mild streptozotocin diabetes in the Göttingen minipig. A novel model of moderate insulin deficiency and diabetes. Am J Physiol 282:E1342-E1351, 2002.
30. Koopmans SJ, Kushwaha RS, DeFronzo RA: Chronic physiologic hyperinsulinemia impairs suppression of plasma free fatty acids and increases de novo lipogenesis but does not cause dyslipidemia in conscious normal rats. Metabolism 48:330-337, 1999.
31. Koopmans SJ, Mandarino L, DeFronzo RA: Time course of insulin action on tissue-specific intracellular glucose metabolism in normal rats. Am J Physiol 274:E642-E650, 1998.
32. Newsholm EA, Leech AR: Biochemistry for the medical sciences. John Wiley & Sons Ltd, 1989.
33. Howard BV, Howard WJ: Dyslipidemia in non-insulin dependent diabetes mellitus. Endocr Rev 15 (3):263-274, 1994.
34. National Cholesterol Education Program: JAMA 285:2486-2497, 2001.
35. Cheng JT, Liu IM, Chi TC, et al: Metformin-like effects of Quei Fu Di Huang Wan, a Chinese herbal mixture, on streptozotocin-induced diabetic rat. Horm Metab Res 33(12):727-732, 2001.
36. Dutta K, Podolin DA, Davidson MB, et al: Cardiomyocyte dysfunction in sucrose-fed rats is associated with insulin resistance. Diabetes 50:1186-1192, 2001.
37. Leng SH, Lu FE, XU LJ: Therapeutic effects of berberine in impaired glucose tolerance rats and its influence on insulin secretion. Acta Pharmacol Sin 25(4):496-502, 2004.
38. Pushparaj PN, Tan BKH, Tan CH: The mechanism of hypoglycaemic action of the semi-purified fractions of Averrhoa bilimbi in streptozotocin-diabetic rats. Life Sciences 70:535-547, 2001.
39. Suzuki M, Odaka H, Suzuki N, et al: Effects of combined pioglitazone and metformin on diabetes and obesity in Wistar fatty rats. Clin Exp Pharmacol Physiol 29(4):269-274, 2002.
40. Koopmans SJ, Frolich M, Gribnau EH, et al: Effect of hyperinsulinemia on plasma leptin concentrations and food intake in rats. Am J Physiol 274:E998-E1001, 1998.
41. Schwartz MW, Figlewicz DP, Baskin DG, et al: Insulin in the brain: a hormonal regulator of energy balance. Endocr Rev 13:387-414, 1992.
42. DeFronzo RA, Ferrannini E: Insulin resistance. A multifaceted syndrome responsible for NIDDM, Obesity, Hypertension, Dyslipidemia, and atherosclerotic cardiovascular disease. Diabetes Care 14(3):173-194, 1991.
43. Lee A, Morley JE: Metformin decreases food consumption and induces weight loss in subjects with obesity with type II non-insulin-dependent diabetes. Obes Res 6:47-53, 1998.
44. Paolisso G, Amato I, Eccellente R, et al: Effect of Metformin on food intake in obese subjects. Eur J Clin Invest 28 :441-446, 1998.

## Claims

1. A method for generating a type II diabetes mellitus and/or Syndrome X model in pigs, comprising partially destructing pancreatic beta-cells in pigs and selecting from said pigs a pig that comprises a fasting plasma glucose level higher than 6 mmol/L or a pig that comprises an elevated fasting plasma level of a functional equivalent of glucose as compared to a fasting plasma level of said functional equivalent of a healthy pig.

2. A method according to claim 1, wherein said pig comprises a fasting plasma glucose level of 10 mmol/L or higher.

3. A method according to claim 1 or claim 2, wherein said pig comprises a fasting plasma glucose level of between 15 and 25 mmol/L.

4. A method according to any one of claims 1-3, wherein said selection comprises measuring fasting plasma glucose levels.

5. A method according to any one of claims 1-4, wherein said selection comprises selecting a pig exhibiting whole body insulin resistance, hepatic insulin resistance and/or plasma triglycerides levels in a fasting and/or postprandial phase elevated by at least a factor of 2 when compared to an untreated pig.

6. A method according to claim 5, wherein said selection further comprises selecting said pig for a non ketotic status or maintenance of growth in the absence of insulin therapy.

7. A method according to any one of claims 1-6, wherein said pig exhibits at least the following features: plasma triglyceride levels in a fasting and/or postprandial phase elevated by at least a factor 2 when compared to an untreated pig and a non-ketotic status.

8. A method according to any one of claims 1-7, wherein said plasma triglyceride level in a fasting phase is higher than 0.8 mmol/L, preferably higher than 1.0 mmol/L.

9. A method according to any one of claims 1-8, wherein said plasma triglyceride level in a postprandial phase is higher than 1.0 mmol/L, preferably higher than 1.2 mmol/L.

10. A method according to any one of claims 1-9, wherein said pig is a diabetes mellitus type II model showing fasting normo- to hyperinsulinemia.

11. A method according to claims 1-4, wherein said pig is a Syndrome X model and is hyperinsulinemic in a fasting and in a postprandial state.

12. A method according to claims 1-11, wherein said partial destruction of said pancreatic beta-cells is achieved by administration of a substance that is preferentially toxic to pancreatic β-cells.

13. A method according to claims 1-12, wherein said substance is streptozotocin (STZ) or alloxan, or a functional equivalent or derivative thereof.

14. A method according to claims 1-13, wherein said STZ or functional equivalent or derivative thereof is made available by infusion or a slow-release formula.

15. A method according to claims 1-4, wherein said infusion lasts at least 2 minutes, preferably 30 minutes.

16. A method according to claims 1-15, wherein said STZ or functional equivalent or derivative thereof is administered in a dosage in the range of 110 to 130 mg/kg to generate a type II diabetes model in pigs.

17. A method according to claims 1-16, wherein said STZ or functional equivalent or derivative thereof is administered in a dosage in the range of 50 to 100 mg/kg to generate a syndrome X model in pigs.

18. A method according to claim 1-17 , wherein said pigs are challenged by environmental factors.

19. A method according to claim 1-18, wherein said pigs have a genetic background for obesity, preferably central obesity.

20. A method according to claims 1-19, wherein said pig has a percentage of fat of at least 15%.

21. A method according to claims 1-20, wherein said pig has a percentage of fat of at least 25%.

22. A pig defined by a weight of 70-150kg at 5-9 months, obtainable by a method according to claims 1-21.

23. A pig according to claims 22, wherein said pig is outbred.

24. A pig according to claims 22 or 23, wherein said pig is a pig of a Pulawska breed or a pig of a Meishan breed.

25. A pig according to claims 22-24, wherein said pig is equipped with at least one catheter in at least one body-compartment.

26. A pig according to claims 22-25, wherein said catheter is permanent.

27. A pig according to claim 26, wherein said catheter is a portal vein catheter.

28. A pig according to claim 27, wherein said portal vein catheter in said pig is inserted via a splenic vein of said pig.

29. A method for inserting a portal vein catheter in a mammal, comprising inserting said catheter via a splenic vein of said mammal.

30. Use of a pig according to claims 22-28 for manipulation of glucose metabolism.

31. Use of a pig according to claims 22-28, wherein said glucose metabolism is insulin-mediated.

32. Use of a pig according to claims 22-28, wherein said manipulation comprises manipulation of type II diabetes mellitus or Syndrome X.

33. Use of a pig according to claims 22-28, for evaluating effects of a treatment on the manifestation of type II diabetes mellitus or syndrome X.

34. Use of a pig according to claim 33, wherein said treatment comprises administration to said pig of nutrition elements or nutrition compounds.

35. Use of a pig according to claims 33 or 34, wherein said treatment comprises administration to said pig of a pharmaceutical composition.

36. Use of a pig according to claims 30-35, wherein said pharmaceutical composition is metformin.

37. Use of a pig according to claims 30-36, for manipulation of metabolic and/or endocrine disorders.

38. Use of a pig according to claims 30-36, for evaluating effects of a pancreatic beta-cell transplant.
